# EUROPEAN PATENT SPECIFICATION

(11) **EP 4 120 937 B1**
(45) Date of publication and mention of the grant of the patent: **07.10.2026**
(21) Application number: 21772491.3
(22) Date of filing: 03.03.2021
(51) Int. Cl.: A61B 17/70, A61B 17/88

(54) **SET SCREW REDUCER FOR MODULAR REDUCTION SCREWS**
STELLSCHRAUBENREDUZIERER FÜR MODULARE REDUKTIONSSCHRAUBEN
RÉDUCTEUR À VIS DE PRESSION POUR VIS DE RÉDUCTION MODULAIRES

(30) Priority: 17.03.2020 US 202016820804
(43) Date of publication of application: 25.01.2023
(73) Proprietor: Warsaw Orthopedic, Inc., Warsaw, IN 46582 (US)
(72) Inventor: BUTLER, Brian A., Memphis, Tennessee 38132 (US); REZACH, William A., Memphis, Tennessee 38132 (US); RICE, Molly K., Memphis, Tennessee 38132 (US)
(74) Representative: Viering, Jentschura & Partner mbB Patent- und Rechtsanwälte
(86) International application number: PCT/US2021/020690
(87) International publication number: WO 2021/188297

(56) References cited:
- EP-B1- 1 585 436
- US-A1- 2011 184 469
- US-A1- 2013 030 445
- US-A1- 2016 128 741
- US-A1- 2017 007 302
- US-A1- 2018 235 677
- US-A1- 2018 368 902
- US-A1- 2019 274 740
- US-A1- 2020 046 410
- US-B2- 8 808 307

## Description

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation, and exercise can be effective, however, may fail to relieve the symptoms associated with these disorders. Surgical treatment of these spinal disorders includes correction, fusion, fixation, discectomy, laminectomy, and/or implantable prosthetics. As part of these surgical treatments, spinal constructs, which include implants such as bone fasteners, connectors, plates, and vertebral rods are often used to provide stability to a treated region. These implants can redirect stresses away from a damaged or defective region while healing takes place to restore proper alignment and generally support the vertebral members. Surgical instruments are employed, for example, to engage the fasteners for attachment to two or more vertebral members. This disclosure describes improvements over these prior technologies.

US 2011 / 184 469 A1 discloses a surgical instrument for reducing a rod toward a bone fastener and securing it thereto with a set screw having a cylindrical fastener engaging member extending between a proximal end and a distal end. The distal portion of said fastener engaging member being adapted to receive a portion of the rod therein and being further adapted to engage the bone fastener. The instrument further being provided with a coaxial set screw installation member extending between a proximal end and a distal end. The distal end of the set screw installation member being adapted to receive and hold the set screw securely within its distal end. The fastener engaging member and coaxial set screw installation member being operable together to reduce the rod distally into the bone fastener and then rotationally attach the set screw into the bone fastener while holding the rod in contact with the bone fastener.

According to US 2013 / 030 445 A1, an instrument assembly includes a downtube having a tubular body with a proximal end and a distal end. In one embodiment, the proximal end forms an opening for receiving a surgical tool, and the distal end forms at least two attachment members for attaching to a bone anchor. The tubular body forms a hollow passage extending from the proximal end to the distal end. The assembly includes a key for insertion into the proximal end of the downtube to attach the downtube to a bone anchor. The key includes an engagement end configured to engage the at least two attachment members when the key is inserted into the downtube. The key is rotatable within the tubular body between a clamping orientation and a releasing orientation.

In US 2017 / 007 302 A1, a method for reducing a fixation rod is provided. The method minimizes the surgical corridor by performing fixation within the surgical corridor defined by the screw extender. The screw extender is attached to the spinal implant. The method includes the steps of coupling the proximal end of a first cannulated body with the screw extender and rotating a second cannulated body within the first cannulated body. The method proceeds to the step of actuating a rod pusher with a first driving feature so as to engage the rod pusher with the fixation rod. The rod pusher includes a bore. The method proceeds to the step of rotating an inserter shaft within first cannulated body, second cannulated body and bore of the rod pusher, the inserter shaft including a proximal end with a second driving feature and a distal end with a setscrew attachment feature.

### SUMMARY

The invention provides a set screw reducer according to claim 1. Further embodiments are described in the dependent claims. The methods/steps described in the present application are not covered by the claims as such, but are helpful for understanding the invention as claimed.

### BRIEF DESCRIPTION OF DRAWINGS

The following drawings are illustrative of particular embodiments of the present disclosure and therefore do not limit the scope of the present disclosure. The drawings are not to scale and are intended for use in conjunction with the explanations in the following detailed description.
FIG. 1 is a perspective view of an illustrative set screw reducer.
FIG. 2 is another perspective view of the illustrative set screw reducer shown in FIG. 1.
FIG. 3 is a perspective view of a distal end of the set screw reducer shown in FIGS. 1-2.
FIG. 4 is a cross-sectional view of the set screw reducer.
FIG. 5 is an illustration of an illustrative external instrument or tool that can be used with the set screw reducer of FIGS. 1-4.
FIG. 6 is a side view of an illustrative set screw that may be used with the set screw reducer of FIGS. 1-4.
FIG. 7 is a cross-sectional view of the illustrative set screw shown in FIG. 6.
FIGS. 8A-8B (collectively referred to herein as "FIG. 8") provide illustrations showing the set screw of FIGS. 6-7 coupled to the set screw reducer of FIGS. 1-4.
FIGS. 9-16 provide illustrations that are useful for understanding how the set screw reducer operates.
FIG. 17 provides a flow diagram of an illustrative method for advancing a set screw in an object (e.g., during a medical procedure).

### DETAILED DESCRIPTION

The following discussion omits or only briefly describes certain conventional features related to surgical systems for treating the spine, which are apparent to those skilled in the art. It is noted that various embodiments are described in detail with reference to the drawings, in which like reference numerals represent like parts and assemblies throughout the several views. Reference to various embodiments does not limit the scope of the claims appended hereto. Additionally, any examples set forth in this specification are intended to be non-limiting and merely set forth some of the many possible embodiments for the appended claims. Further, particular features described herein can be used in combination with other described features in each of the various possible combinations and permutations.

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

Embodiments of the present disclosure relate generally, for example, to medical devices and methods for treating musculoskeletal disorders, and more particularly, to surgical systems and methods for treating the spine. Embodiments of the devices, methods, and systems are described below with reference to the Figures. Here, the methods/steps described below are not covered by the claims as such, but are helpful for understanding the invention as claimed.

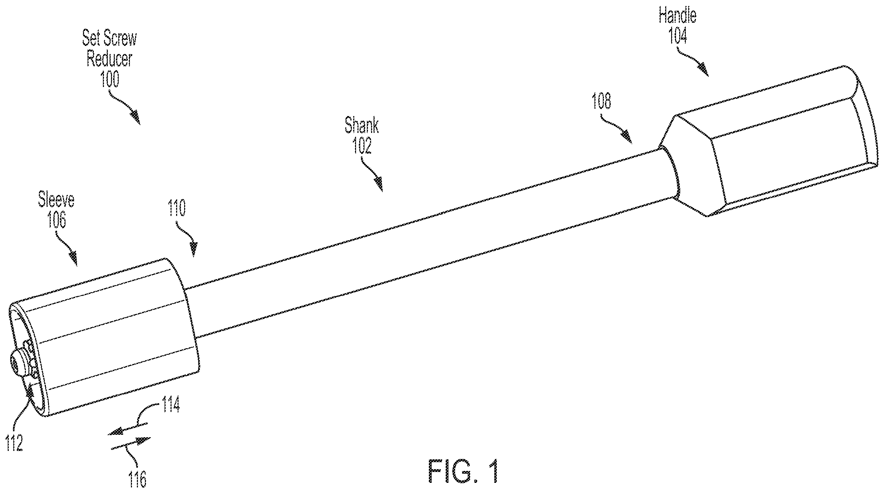

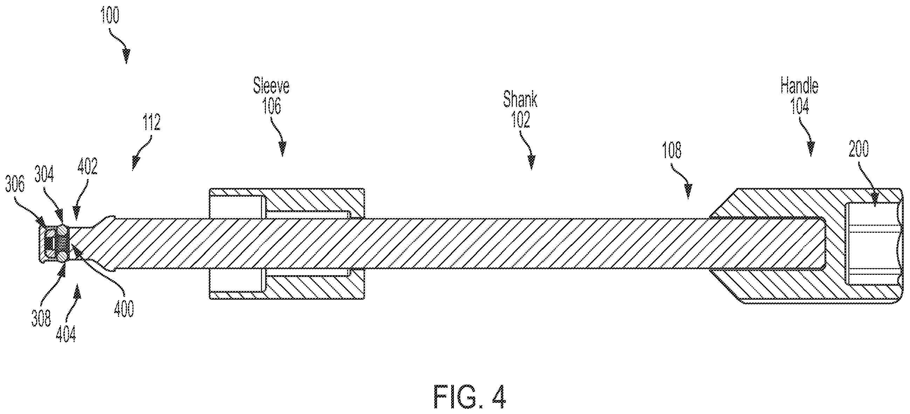

FIGS. 1-3 provide perspective views of a set screw reducer 100. A cross-sectional view of the set screw reducer is provided in FIG. 4. As shown in FIGS. 1-4, the set screw reducer **100** comprises a shank **102** and a handle **104.** The handle **104** resides at a proximal end **108** of the shank **102.** The shank **102** and handle **104** may be integrally formed as one part (not shown), or alternatively be coupled to each other via a coupling means as shown in FIG. 4. The coupling means can include, but is not limited to, a weld, adhesive, and/or threads. The shank **102** and handle **104** are formed of stainless steel, titanium or other alloy which is resistant to corrosion. The handle **104** has a size and shape that allows an individual to easily grip and turn the same without discomfort. The handle **104** transfers torque from the individual to the shank **102.**

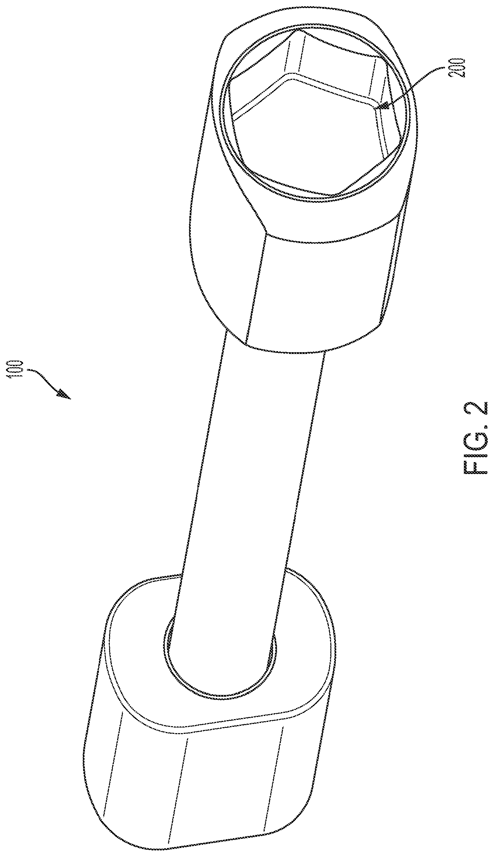

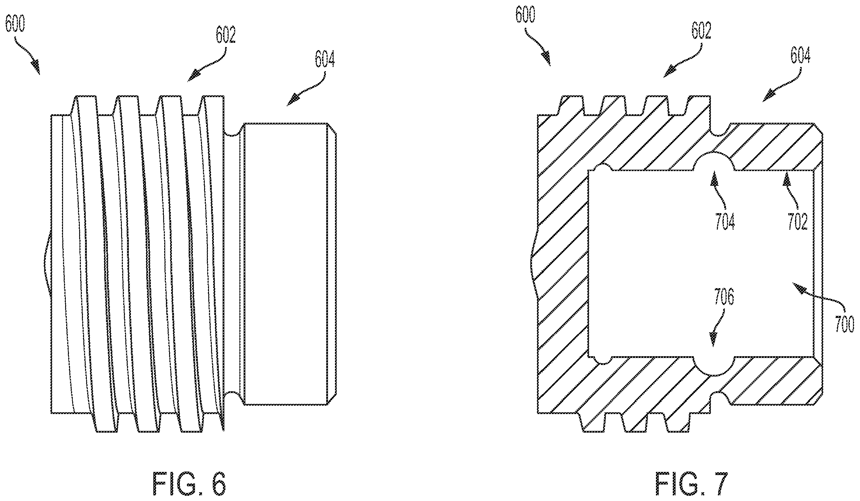

Notably, the handle **104** has a socket **200** formed therein as shown in FIG. 2 and FIG. 4. The socket **200** is sized and shaped to receive an end of an external instrument or tool (e.g., a power attachment, or a manual wrench) that can facilitate the rotation of the shank **102.** The external instrument or tool may optionally be employed when it becomes difficult for the individual to apply torque to the set screw reducer **100** when driving a screw (e.g., such as screw **600** shown in FIGS. 6-7) into an object (e.g., a bone, an implant or a receiver during a medical procedure).

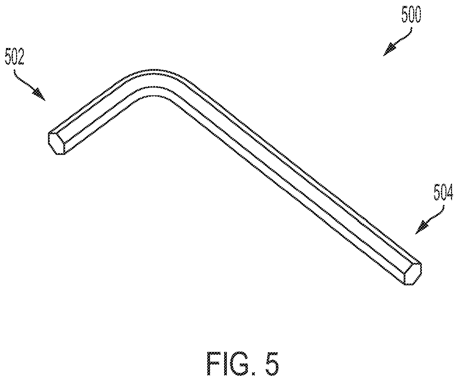

The external instrument or tool can include, but is not limited to, a power attachment with a hex bit, or a manual Allen wrench. Power attachments and Allen wrenches are well known in the art, and therefore will not be described here. For example, an illustrative Allen wrench is shown in FIG. 5. In the Allen wrench scenario, the socket **200** has a hexagonal shape and the Allen wrench **500** has a hexagonal head at each end **502, 504** which can be inserted into the hexagonal shaped socket **200** of the set screw reducer **100.** A torque may be applied to the set screw reducer **100** by the Allen wrench **500,** whereby the set screw reducer is caused to rotate. The set screw reducer **100** transfers the torque to the set screw (e.g., set screw **600** of FIGS. 6-7), whereby the set screw is caused to rotate such that the set screw is advanced into or driven out of an object (e.g., a bone, an implant, or a receiver during a medical procedure). The present solution is not limited to the particulars of this example.

Set screws are well known in the art, and therefore will not be described herein. Any known or to be known set screw can be used herein without limitation. Still, an illustrative set screw **600** is shown in FIGS. 6-7. The features of set screw **600** will become apparent as the discussion progresses.

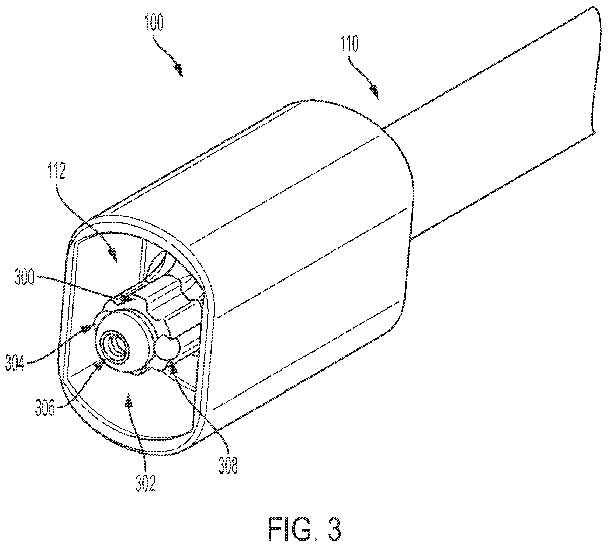

The set screws that are used with the set screw reducer **100** may be of the Phillip type having cross, hexalobe or star-shaped recesses in their heads. In this regard, a distal end **110** of the shank **102** has a tip **112** that is sized and shaped to fit in the cross, hexalobe or star-shaped recesses of the set screws. A perspective view of an illustrative architecture for the tip **112** of shank **102** is provided in FIG. 3. As shown in FIG. 3, the tip **112** has a star-shape in which a plurality of protrusions **300** are provided for engaging recess sidewalls of the set screws. This engagement between the tip **112** and a set screw allows torque to be transferred from the set screw reducer **100** to the set screw.

The tip **112** also comprises a means **302** to frictionally engage a set screw (e.g., set screw **600** of FIGS. 6-7) such that: the set screw remains coupled to the set screw reducer **100** prior to thread engagement between a threaded section (e.g., section **602** of FIGS. 6-7) of the set screw and the object, and during the driving of the set screw into the object; a break-off section (e.g., section **604** of FIGS. 6-7) of the set screw remains coupled to the set screw reducer **100** while being broken apart from the threaded section of the set screw via an application of torque thereto, and while the set screw reducer **100** is being removed away from the object; and the break-off section (e.g., section **604** of FIGS. 6-7) of the set screw can be released from the set screw reducer **100** after the set screw reducer has been moved away from the object. The frictional retention means can include, but is not limited to, a tapered hex shaped tip **112,** at least one resiliently biased engagement part (e.g., a ball bearing), and/or a rough surface provided on the tip **112.**

The set screw reducer **100** is shown in FIGS. 1-4 as comprising a frictional retention means including resiliently biased engagement parts. In this architecture, the engagement parts comprise ball bearings **304, 308** that are aligned with each other and disposed on opposing sides **402, 404** of the shank's tip **112.** The ball bearings **304, 308** are disposed in the shank's tip **112** so that the ball bearings **304, 308** can slide into and out of the shank's tip **112.** A resilient member **400** is disposed between the two ball bearings **304, 308.** The resilient member **400** is normally in an uncompressed state, and therefore resiliently biases the ball bearings **304, 308** away from each other as shown in FIG. 4. The resilient member **400** can include, but is not limited to, a coil or spring. The ball bearings **304, 308** and resilient member **400** are held in position relative to each other by a cap **306.**

When the shank's tip **112** of the set screw reducer **100** is being slid into a socket of a set screw (e.g., set screw **600** of FIGS. 6-7), the ball bearings **304, 308** slide against a sidewall (e.g., sidewall **702** of FIG. 7) of a socket (e.g., socket **700** of FIGS. 6-7) formed in the set screw. This sliding engagement causes the ball bearings **304, 308** to move towards each other and into the shank's tip **112.** In effect, the resilient member **400** is compressed by the ball bearings **304, 308.** When the ball bearings **304, 308** become aligned with detents (e.g., detents **704, 706** of FIG. 7), a groove or an interior circumferential undercut of the set screw, the resilient member **400** resiliently biases the ball bearings **304, 308** into the detents **704, 706.** At this time, a frictional engagement is provided between the set screw reducer **100** and the set screw.

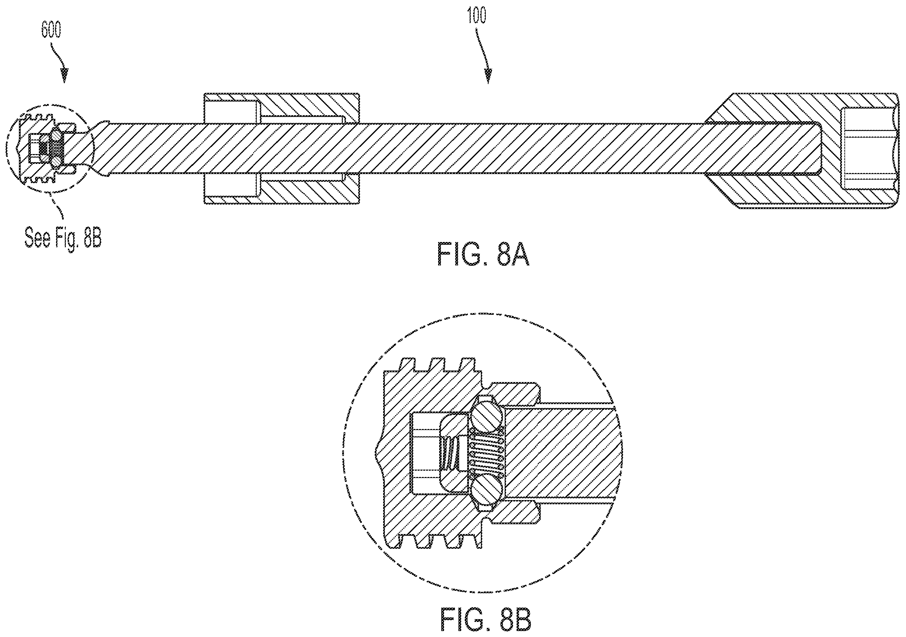

An illustration showing the ball bearings **304, 308** of the set screw reducer **100** disposed in detents **704, 706** of a set screw **600** is provided in FIG. 8. The present solution is not limited to this particular type of frictional means. Other frictional engagement solutions may alternatively or additionally be used as noted above.

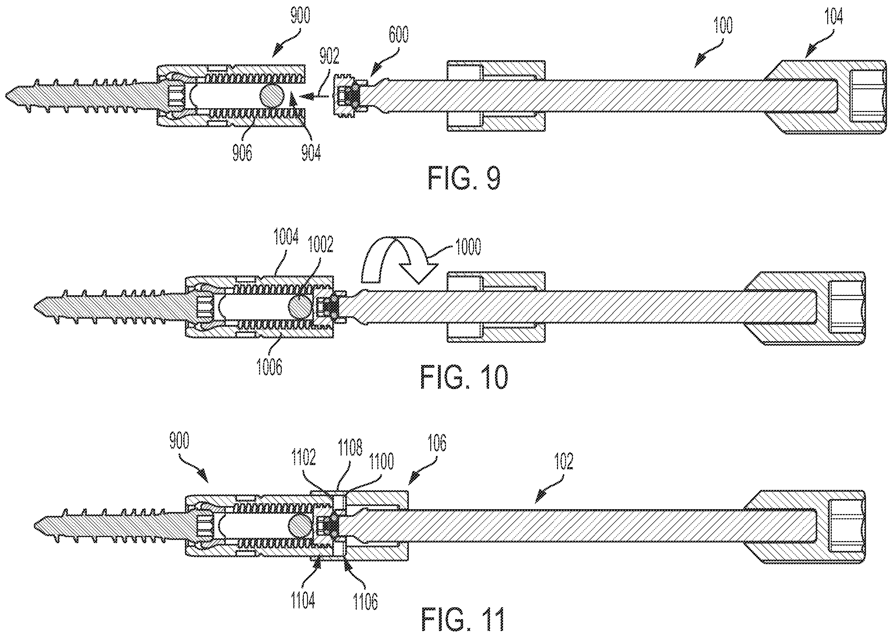

As shown in FIGS. 1-4, the set screw reducer **100** also comprises a sleeve **106** slidingly disposed on the shank **102.** The sleeve **106** is able to slide in two opposing directions **114, 116** along an elongate length of the shank **102.** This sliding ability of the sleeve **106** ensures that the sleeve **106** does not obstruct, prevent and/or interfere with the coupling of the set screw to the set screw reducer **100.** In this regard, it should be understood that the sleeve **106** may be slid in direction **116** towards handle **104** prior to when the set screw reducer's operational tip **112** is inserted into the socket/recess of the set screw. In this way, the sleeve **106** is moved to a position (e.g., the position shown in FIG. 9) in which (i) the sleeve does not cover or otherwise block an individual's ability to view tip **112** and/or (ii) a possibility of the sleeve interfering with a coupling between the set screw reducer and the set screw is decreased or eliminated. The sleeve **106** may be maintained in this position while a frictional engagement between the set screw reducer **100** and the set screw is being established.

Notably, the sleeve **106** is provided to prevent or substantially prevent splaying (or spreading outward) of a receiver (e.g., receiver **900** of FIG. 9) while the set screw (e.g., set screw **600** of FIGS. 6-7) is being driven into the same via the set screw reducer **100.** Illustrations showing the set screw reducer being used to drive a set screw into a receiver are provided in FIGS. 9-16.

As shown in FIG. 9, a set screw **600** is frictionally coupled to the set screw reducer 100 in the manner described above. As such, the components **100, 600** are simultaneously or concurrently moved in a direction **902** towards the receiver **900** during a set screw driving process as also shown in FIG. 9.

In some scenarios, a sleeve **106** of the set screw reducer **100** may slidingly move in direction **902** towards receiver **900** along the elongate length of the shank **102** while the components **100, 600** are being moved towards the receiver **900.** The sliding movement of the sleeve **106** may be caused by a gravitational force being applied thereto. The present solution is not limited in this regard. For example, the sleeve **106** could be held (e.g., manually or via a mechanical means (e.g., a depressible post of the shank)) at a certain location on shank **102** until a later time in the process as discussed below.

Once the set screw **600** is aligned and in contact with a threaded hole **904** of the receiver **900,** an individual applies a rotational force to the set screw reducer **100** (e.g., directly via handle **104** or indirectly via use of an external instrument or tool). This rotational force is applied by rotating the set screw reducer **100** in a counter clockwise direction **1000** (not shown) or alternatively in a clockwise direction (shown in FIG. 10). This rotational force or torque is transferred from the set screw reducer **100** to the set screw **600,** and causes a threaded portion **602** of the set screw to threadingly engage threads **906** of the receiver **900.**

Rotation of the set screw **600** is continued as shown in FIG. 10 so that the set screw **600** abuts a bar, rod or post **1002** that is at least partially inserted through channels formed between sidewalls **1004, 1006** of the receiver **900.** Thereafter, the sleeve **106** is slidingly moved in direction **902** towards receiver **900** along the elongate length of the shank **102.** The sliding movement of the sleeve **106** may be caused manually by the individual or caused by a gravitational force being applied thereto. The sleeve **106** is slid in direction **902** until either (i) its internal engagement surface **1100** abuts an engagement surface **1102** of the receiver **900** or (ii) stop structures (not shown in FIGS. 9-16) (e.g., one or more protruding structures) of the shank **102** prevent further movement of the sleeve **106** in direction **902.** In either case, top portions **1104** of the receiver's sidewalls **1004, 1006** are received in a recess **1106** of the sleeve **106,** as shown in FIG. 11. Consequently, the receiver's top portions **1104** are enclosed by a sidewall **1108** of the sleeve **106.** Notably, the sleeve sidewall **1108** prevents or substantially prevents the splaying of the receiver's top portions **1104** by the set screw reducer **100** as the set screw is being advanced through the threaded hole **904,** as will become apparent with the following discussion.

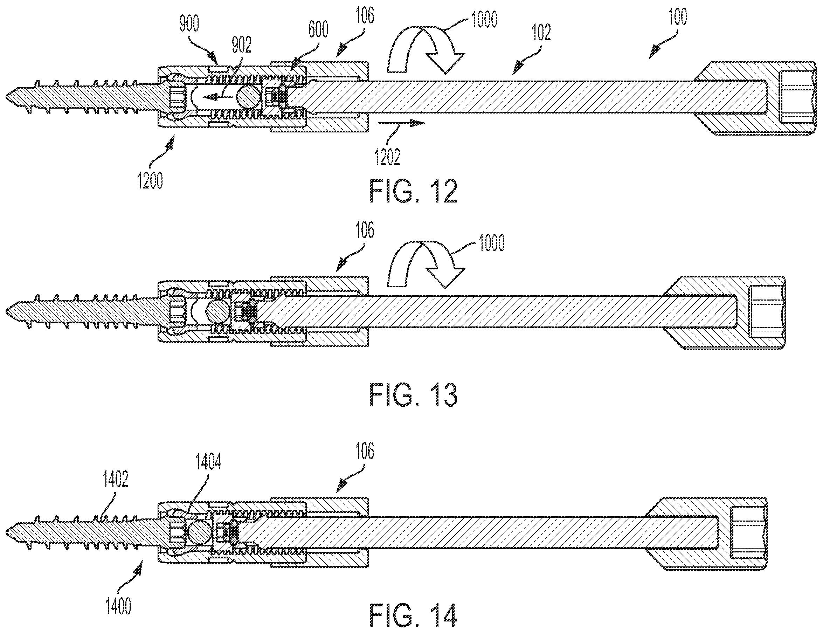

As shown in FIGS. 12-14, the set screw reducer **100** is continuously rotated so as to advance the set screw **600** through a threaded hole **904** of the receiver **900.** During this set screw advancement, the set screw **600** applies a pushing force in direction **902** directly to the bar/rod/post **1002** as it is being advanced through the threaded hole **904.** This pushing force causes the bar/rod/post **1002** to travel in direction **902** along with the set screw **600** towards a distal end **1200** of the receiver **900.**

Also during the set screw advancement process, the receiver **900** applies a pushing force on the sleeve **106** such that the sleeve slides in direction **1202** along the elongate length of the shank **102.** In effect, the receiver's top portions **1104** are continuously disposed in the sleeve **106** while the set screw is being advanced through the threaded hole **904,** as shown in FIGS. 12-14. Consequently, the sleeve **106** provides a means to resist the outward splaying of the receiver's top portions **1104** with the set screw reducer **100** throughout the set screw advancement process, and thereby also facilitates an increase speed and efficiency of the set screw advancement process.

As shown in FIG. 14, the set screw **600** is then tightened against the bar/rod/post **1002** to prevent the bar/rod/post from moving relative to the object **1400** (e.g., a screw **1402** with a crown **1404).** The set screw **600** exerts a compression or clamping force to the bar/rod/post **1002.** In this way, the bar/rod/post **1002** is securely retained in a given position relative to the object **1400** by the set screw **600** and the receiver **900.**

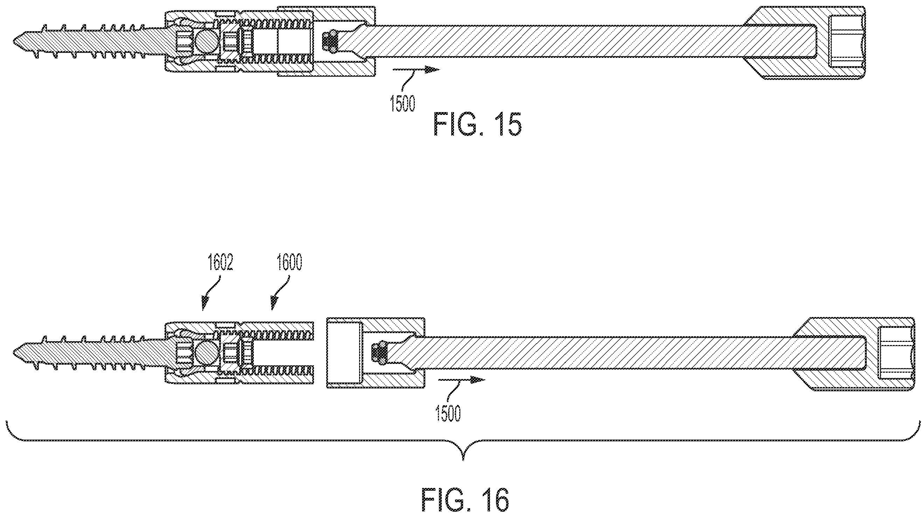

Once the set screw **600** is tightened against the bar/rod/post **1002,** the set screw reducer **100** is removed from the receiver **900,** as shown by FIGS. 15-16. In this regard, a pulling force is applied to the set screw reducer **100** in direction **1500.** This pulling force causes the frictional engagement between the set screw reducer **100** and set screw **600** to be discontinued, i.e., the set screw reducer's tip **112** is pulled out of the recess/socket of the set screw **600.**

Another tool (not shown) may then be used to apply torque to the set screw so as to (i) cause the break-off section **604** of the set screw to break away from the threaded section **602** of the set screw, and (ii) remove the break-off section **604** of the set screw from the threaded hole **904** of the receiver **900.** The present solution is not limited in this regard. Alternatively, the set screw reducer **100** is used instead of the additional tool for performing actions (i) and (ii).

Yet another tool may be used to apply a lateral force to the receiver **900** so as to (i) cause the break-off section **1600** of the receiver to break away from the retainer section **1602** of the receiver, and (ii) move the break-off section **1600** away from the object.

Notably, during some medical procedures, a plurality of set screws may need to be advanced through a plurality of receivers. A plurality of set screw reducers **100** can be concurrently used in such scenarios to advance the set screws through receivers at the same time or in an alternating manner during a single process. For example, a first set screw reducer is used to advance a first set screw by a first amount during a first time period, and a second set screw reducer is used to advance a second set screw by a second amount during a second subsequent time period, where the first and second amounts are the same or different. This process is repeated until the first and second set screws are advanced by a desired amount into one or more objects. The present solution is not limited to the particulars of this example.

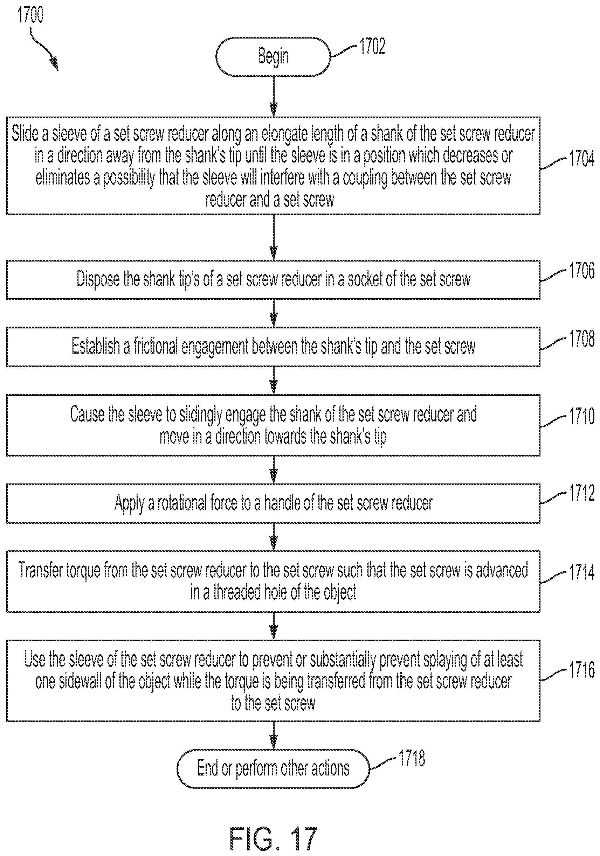

Referring now to FIG. 17, there is provided a flow diagram of an illustrative method **1700** for advancing a set screw (e.g., set screw **600** of FIG. 6-7) in an object (e.g., receiver **900** of FIGS. 9-16) (e.g., during a medical procedure). Method **1700** begins with **1702** and continues with **1704** where a sleeve (e.g., sleeve **106** of FIGS. 1-4) of a set screw reducer (e.g., set screw reducer **100** of FIGS. 1-4) is slid along an elongate length of a shank (e.g., shank **102** of FIGS. 1-4) of the set screw reducer in a direction (e.g., direction **116** of FIG. 1) away from the shank's tip (e.g., tip **112** of FIG. 1) until the sleeve is in a positon (e.g., the position shown in FIG. 9) which decreases or eliminates the sleeve's interference with a coupling between the set screw reducer and the set screw. Notably, the sleeve is integrated with the operational parts (e.g., handle **104** and shank **102** of FIGS. 1-4) of the set screw reducer such that the set screw reducer has multiple purposes. These purposes include, but are not limited to, advancing a set screw into an object, removing a set screw from an object, and/or preventing splaying of at least one sidewall of the object while the torque is being transferred from the set screw reducer to the set screw.

Next in **1706,** the shank's tip is disposed in a socket (e.g., socket **700** of FIGS. 6-7) of the set screw. A frictional engagement is established in **1708** between the shank's tip and the set screw. In some scenarios, the frictional engagement is established by resiliently biasing at least one engagement part of the set screw reducer into a detent or a groove (e.g., detent or groove **704, 706** of FIG. 7) formed in a sidewall (e.g., sidewall **702** of FIG. 7) of the set screw's socket. The at least one engagement part can include, but is not limited to, a ball bearing (e.g., ball bearing **304, 308** of FIG. 3) that is resiliently biased by a resilient member (e.g., resilient member **400** of FIG. 4) in a direction out and away from the shank tip. The present solution is not limited to the particulars of this scenario.

In **1710,** the sleeve is caused to slidingly engage the shank of the set screw reducer and move in a direction (e.g., direction **114** of FIG. 1) towards the shank's tip. A rotational force is then applied in **1712** to the handle of the set screw reducer. The rotational force can be applied with or without the assistance of an external instrument or tool (e.g., wrench **500** of FIG. 5). In this regard, it should be understood that an end (e.g., end **502, 504** of FIG. 5) of the external instrument or tool can be received in a socket (e.g., socket **200** of FIG. 2) formed in the handle of the set screw reducer, and thereafter used to cause rotation of the handle and shank of the set screw reducer.

**In 1714,** torque is transferred from the set screw reducer to the set screw such that the set screw is advanced in a threaded hole (e.g., threaded hole **904** of FIG. 9) of the object. The torque is transferred from the set screw reducer to the set screw in response to a rotational force being applied to a handle of the set screw reducer.

In **1716,** the sleeve of the set screw reducer is used to prevent or substantially prevent splaying of at least one sidewall (e.g., sidewall **1004, 1006** of FIG. 10) of the object while the torque is being transferred from the set screw reducer to the set screw. As part of splaying prevention, the sleeve resists an outward bending of the at least one sidewall of the object throughout the advancement of the set screw in the object. In this regard, it should be understood that: a free end (e.g., end **1104** of FIG. 11) of the at least one sidewall of the object is continuously disposed within a recess (e.g., recess **1106** of FIG. 11) formed in the sleeve, while the set screw reducer is being used to advance the set screw in the object; and the free end of the at least one sidewall of the object causes the sleeve to move along an elongate length of the shank in a direction away from the shank tip while the set screw is being advanced in the object. Subsequently, **1718** is performed where method **1700** ends or other actions are performed.

## Claims

1. A set screw reducer (100), comprising:
a shank (102) having a shank tip (112) sized and shaped to fit in a socket (700) of a set screw (600); and
a sleeve (106) disposed on the shank (102) such that the sleeve (106) slidingly engages the shank (102) and is movable at least in a first direction towards the shank tip (112);
wherein torque is transferable from the shank (102) to the set screw (600); and
wherein the sleeve (106) prevents splaying of at least one sidewall (1004, 1006) of a receiver (900) for a bar, a rod or a post (1002) while the torque is being transferred from the shank (102) to the set screw (600),
the set screw reducer (100) further comprising a frictional retention structure that establishes a frictional engagement between the shank tip (112) and the set screw (600),
wherein the frictional engagement is established by resiliently biasing at least one engagement part of the frictional retention structure into a detent or a groove formed in a sidewall (702) of the socket (700) of the set screw (600),
**characterized in that**
the at least one engagement part comprises ball bearings (304, 308) that are aligned with each other and disposed on opposing sides (402, 404) of the shank tip (112),
the ball bearings (304, 308) are disposed in the shank tip (112) so that the ball bearings (304, 308) can slide into and out of the shank tip (112),
a resilient member (400) is disposed between the ball bearings (304, 308), and
the resilient member (400), in an uncompressed state, resiliently biases the ball bearings (304, 308) away from each other.

2. The set screw reducer (100) according to claim 1, wherein the sleeve (106) is slidable along an elongate length of the shank (102) in a direction away from the shank tip (112) until the sleeve (106) is in a position which substantially eliminates the sleeve interference with a coupling between the surgical instrument (100) and the set screw (600).

3. The set screw reducer (100) according to claim 1, wherein the torque is transferrable from the shank (102) to the set screw (600) in response to a rotational force being applied to a handle (104) of the set screw reducer (100).

4. The set screw reducer (100) according to claim 1, wherein the sleeve (106), as part of splaying prevention, resists an outward bending of the at least one sidewall (1004, 1006) of the receiver (900) throughout the advancement of the set screw (600) in the receiver (900).

5. The set screw reducer (100) according to claim 4, wherein a free end (1104) of the at least one sidewall (1004, 1006) of the receiver (900) is continuously disposed within a recess (1106) formed in the sleeve (106), while the set screw reducer (100) is being used to advance the set screw (600) in the receiver (900),
wherein, optionally, the free end (1104) of the at least one sidewall (1004, 1006) of the receiver (900) causes the sleeve (106) to move along an elongate length of the shank (102) in a direction away from the shank tip (112) while the set screw (600) is being advanced in the receiver (900).

6. The set screw reducer (100) according to claim 1, further comprising a handle (104) having a socket (200) to receive an end of an external instrument or tool that facilitates rotation of the handle (104) during advancement of the set screw (600) in the receiver (900).

## Patentansprüche

1. Stellschrauben-Reduzierstück (100), umfassend:
einen Schaft (102) mit einer Schaftspitze (112), die bemessen und geformt ist, um in eine Fassung (700) einer Stellschraube (600) zu passen; und
eine Hülse (106), die auf dem Schaft (102) derart angeordnet ist, dass die Hülse (106) mit dem Schaft (102) gleitend in Eingriff steht und mindestens in einer ersten Richtung zu der Schaftspitze (112) hin beweglich ist;
wobei Drehmoment von dem Schaft (102) auf die Stellschraube (600) übertragbar ist; und
wobei die Hülse (106) das Aufspreizen mindestens einer Seitenwand (1004, 1006) einer Aufnahme (900) für eine Stange, einen Stab oder einen Pfosten (1002) verhindert, während das Drehmoment von dem Schaft (102) auf die Stellschraube (600) übertragen wird,
das Stellschrauben-Reduzierstück (100) ferner umfassend eine Reibungshaltestruktur, die einen Reibeingriff zwischen der Schaftspitze (112) und der Stellschraube (600) herstellt,
wobei der Reibeingriff durch elastisches Vorspannen mindestens eines Eingriffsteils der Reibhaltestruktur in eine Arretierung oder eine Nut hergestellt wird, die in einer Seitenwand (702) der Fassung (700) der Stellschraube (600) ausgebildet ist,
**dadurch gekennzeichnet, dass**
das mindestens eine Eingriffsteil umfasst Kugellager (304, 308), die zueinander ausgerichtet sind und auf gegenüberliegenden Seiten (402, 404) der Schaftspitze (112) angeordnet sind,
die Kugellager (304, 308) in der Schaftspitze (112) angeordnet sind, sodass die Kugellager (304, 308) in die Schaftspitze (112) hinein und aus dieser heraus gleiten können,
ein elastisches Element (400) zwischen den Kugellagern (304, 308) angeordnet sind, und
das elastische Element (400), in einem unkomprimierten Zustand, die Kugellager (304, 308) elastisch voneinander weg vorspannt.

2. Stellschrauben-Reduzierstück (100) nach Anspruch 1, wobei die Hülse (106) entlang einer langgestreckten Länge des Schafts (102) in einer Richtung weg von der Schaftspitze (112) verschiebbar ist, bis sich die Hülse (106) in einer Position befindet, die die Störung durch die Hülse bei einem Koppeln zwischen dem chirurgischen Instrument (100) und der Stellschraube (600) im Wesentlichen eliminiert.

3. Stellschrauben-Reduzierstück (100) nach Anspruch 1, wobei das Drehmoment von dem Schaft (102) auf die Stellschraube (600) in Reaktion auf eine Drehkraft, die auf einen Griff (104) des Stellschrauben-Reduzierstücks (100) ausgeübt wird, übertragbar ist.

4. Stellschrauben-Reduzierstück (100) nach Anspruch 1, wobei die Hülse (106) als Teil einer Abspreizverhinderung einem Auswärtsbiegen der mindestens einen Seitenwand (1004, 1006) der Aufnahme (900) während des gesamten Vorschubs der Stellschraube (600) in der Aufnahme (900) entgegenwirkt.

5. Stellschrauben-Reduzierstück (100) nach Anspruch 4, wobei ein freies Ende (1104) der mindestens einen Seitenwand (1004, 1006) der Aufnahme (900) kontinuierlich innerhalb einer Aussparung (1106), die in der Hülse (106) ausgebildet ist, angeordnet ist, während das Stellschrauben-Reduzierstück (100) verwendet wird, um die Stellschraube (600) in der Aufnahme (900) vorzuschieben,
wobei optional das freie Ende (1104) der mindestens einen Seitenwand (1004, 1006) der Aufnahme (900) bewirkt, dass sich die Hülse (106) entlang einer langgestreckten Länge des Schafts (102) in einer Richtung weg von der Schaftspitze (112) bewegt, während die Stellschraube (600) in der Aufnahme (900) vorgeschoben wird.

6. Stellschrauben-Reduzierstück (100) nach Anspruch 1, ferner umfassend einen Griff (104), der eine Fassung (200) aufweist, um ein Ende eines externen Instruments oder Werkzeugs aufzunehmen, das die Drehung des Griffs (104) während des Vorschiebens der Stellschraube (600) in die Aufnahme (900) ermöglicht.

## Revendications

1. Réducteur à vis de réglage (100), comprenant :
une tige (102) ayant une pointe de tige (112) dimensionnée et profilée pour s'insérer dans une douille (700) d'une vis de réglage (600) ; et
un manchon (106) disposé sur la tige (102) de telle sorte que le manchon (106) vient en prise de manière coulissante avec la tige (102) et est mobile au moins dans une première direction vers la pointe de tige (112) ;
dans lequel le couple peut être transféré de la tige (102) à la vis de réglage (600) ; et
dans lequel le manchon (106) empêche l'évasement d'au moins une paroi latérale (1004, 1006) d'un récepteur (900) pour une barre, une tige ou un poteau (1002) pendant que le couple est transféré de la tige (102) à la vis de réglage (600),
le réducteur à vis de réglage (100) comprenant en outre une structure de rétention par friction qui établit une mise en prise par friction entre la pointe de tige (112) et la vis de réglage (600),
dans lequel la mise en prise par friction est établie en sollicitant élastiquement au moins une partie de mise en prise de la structure de rétention par friction dans un cran ou une rainure formée dans une paroi latérale (702) de la douille (700) de la vis de réglage (600),
**caractérisé en ce que**
l'au moins une partie de mise en prise comprend des roulements à billes (304, 308) qui sont alignés les uns sur les autres et disposés sur des côtés opposés (402, 404) de la pointe de tige (112),
les roulements à billes (304, 308) sont disposés dans la pointe de tige (112) de sorte que les roulements à billes (304, 308) peuvent coulisser dans et hors de la pointe de tige (112),
un élément élastique (400) est disposé entre les roulements à billes (304, 308), et
l'élément élastique (400), dans un état non comprimé, sollicite élastiquement les roulements à billes (304, 308) pour les éloigner les uns des autres.

2. Réducteur à vis de réglage (100) selon la revendication 1, dans lequel le manchon (106) peut coulisser le long d'une longueur allongée de la tige (102) dans une direction à l'écart de la pointe de tige (112) jusqu'à ce que le manchon (106) soit dans une position qui élimine sensiblement l'interférence du manchon avec un accouplement entre l'instrument chirurgical (100) et la vis de réglage (600).

3. Réducteur à vis de réglage (100) selon la revendication 1, dans lequel le couple peut être transféré de la tige (102) à la vis de réglage (600) en réponse à une force de rotation appliquée à une poignée (104) du réducteur à vis de réglage (100).

4. Réducteur à vis de réglage (100) selon la revendication 1, dans lequel le manchon (106), dans le cadre de la prévention de l'évasement, résiste à une flexion vers l'extérieur de l'au moins une paroi latérale (1004, 1006) du récepteur (900) tout au long de l'avancement de la vis de réglage (600) dans le récepteur (900).

5. Réducteur à vis de réglage (100) selon la revendication 4, dans lequel une extrémité libre (1104) de l'au moins une paroi latérale (1004, 1006) du récepteur (900) est disposée de manière continue à l'intérieur d'un évidement (1106) formé dans le manchon (106), tandis que le réducteur à vis de réglage (100) est utilisé pour faire avancer la vis de réglage (600) dans le récepteur (900),
dans lequel, éventuellement, l'extrémité libre (1104) de l'au moins une paroi latérale (1004, 1006) du récepteur (900) amène le manchon (106) à se déplacer le long d'une longueur allongée de la tige (102) dans une direction à l'écart de la pointe de tige (112) tandis que la vis de réglage (600) est avancée dans le récepteur (900).

6. Réducteur à vis de réglage (100) selon la revendication 1, comprenant en outre une poignée (104) ayant une douille (200) pour recevoir une extrémité d'un instrument ou d'un outil externe qui facilite la rotation de la poignée (104) pendant l'avancement de la vis de réglage (600) dans le récepteur (900).
